# EUROPEAN PATENT SPECIFICATION

(11) **EP 2 254 573 B1**
(45) Date of publication and mention of the grant of the patent: **16.05.2012**
(21) Application number: 09712828.4
(22) Date of filing: 20.02.2009
(51) Int. Cl.: A61K 31/357, A61K 31/352, A61K 31/05, A61K 31/716, A61K 45/06, A61P 1/00, A61P 35/00

(54) **COMPOSITIONS COMPRISING PHYTOESTROGENS SELECTIVE FOR ESTROGEN BETA RECEPTOR AND DIETARY FIBRES**
ZUSAMMENSETZUNGEN MIT FÜR ÖSTROGEN-BETA-REZEPTOR SELEKTIVEN PHYTOÖSTROGENEN UND NAHRUNGSFASERN
COMPOSITIONS COMPRENANT DES PHYTOESTROGÈNES SÉLECTIFS POUR LE RÉCEPTEUR BÊTA DE L'ESTROGÈNE ET DES FIBRES ALIMENTAIRES

(30) Priority: 22.02.2008 IT BA20080008
(43) Date of publication of application: 01.12.2010
(73) Proprietor: CM&D Pharma Limited, Esher Surrey KT10 9AD (GB)
(72) Inventor: DI LEO, Alfredo, I-70043 Monopoli (IT); BARONE, Michele, I-70124 Bari (IT)
(74) Representative: Gervasi, Gemma
(86) International application number: PCT/EP2009/052038
(87) International publication number: WO 2009/103788

(56) References cited:
- EP-A- 0 846 704
- DE-A1-102004 054 133
- TAMURA MOTOI ET AL: "Effects of soy oligosaccharides on plasma and cecal isoflavones, and cecal enzyme activities in mice" JOURNAL OF NUTRITIONAL SCIENCE AND VITAMINOLOGY, UNIVERSITY OF TOKYO PRESS, TOKYO, JP, vol. 49, no. 3, 1 June 2003 (2003-06-01), pages 168-171, XP009117194 ISSN: 0301-4800
- STANGROOM K E ET AL: "Effect of whole and fractionated dietary alfalfa meal on zearalenone toxicosis and metabolism in rats and swine" CANADIAN JOURNAL OF PHYSIOLOGY AND PHARMACOLOGY, NRC RESEARCH PRESS, CANADA, vol. 62, no. 9, 1 September 1984 (1984-09-01), pages 1219-1224, XP009117192 ISSN: 0008-4212
- HOH CARMEN S I ET AL.: "A clinical pilot study of oral silibinin, a putative colorectal cancer preventive agent, in patients with primary colorectal cancer and colorectal liver metastases" PROCEEDINGS OF THE AMERICAN ASSOCIATION FOR CANCER RESEARCH ANNUAL MEETING, vol. 47, April 2006 (2006-04), page 752, XP001537130
- MAHYAR-ROEMER MOJGAN ET AL: "Role of Bax in resveratrol-induced apoptosis of colorectal carcinoma cells" BMC CANCER, BIOMED CENTRAL, LONDON, GB, vol. 2, no. 1, 17 October 2002 (2002-10-17), page 27, XP021016050 ISSN: 1471-2407
- LECHNER DANIEL; CROSS HEIDE S: "Genistein prevents downregulation of 25-D-3-1 alpha-hydroxylase by 1,25-(OH)(2)-D-3 in human mammary and colorectal tumor cells: Relevance for cancer prevention" PROCEEDINGS OF THE AMERICAN ASSOCIATION FOR CANCER RESEARCH ANNUAL MEETING, vol. 45, March 2004 (2004-03), page 736, XP001539466
- HILLMAN L; PETERS S; FISHER A; POMARE E W: "DIFFERING EFFECTS OF PECTIN CELLULOSE AND LIGNIN ON STOOL PH TRANSIT TIME AND WEIGHT" BRITISH JOURNAL OF NUTRITION, vol. 50, no. 2, 1983, pages 189-196, XP002529043
- ANONYMOUS: 'Phytoestrogen', [Online] National Cancer Institute - Dictionary of Cancer Terms Retrieved from the Internet: <URL:http://www.cancer.gov/dictionary?CdrID =330175> [retrieved on 2009-12-21]
- SANDBORN W. J.: "Oral 5-ASA therapy in ulcerative colitis", J. CLIN. GASTROENTEROL., vol. 42, no. 4, April 2008 (2008-04), pages 338-344,
- KOUTROUBAKIS I. E.: "Recent advances in the management of distal ulcerative colitis", WORLD J. GASTROINTEST. PHARMACOL. THER., vol. 1, no. 2, 6 April 2010 (2010-04-06), pages 43-50,
- MAJEWSKI M. ET AL.: "Efficacy of rifaximin, a nonabsorbed oral antibiotic, in the treatment of small intestinal bacterial overgrowth", AM. J. MED. SCI., vol. 333, no. 5, May 2007 (2007-05), pages 266-270,

## Description

### FIELD OF INVENTION

The present invention relates to compositions comprising one or more phytoestrogens in association with dietary fibres, insoluble and resistant to enzymatic digestion, and to the use of said compositions for the prophylactic and/or therapeutic treatment of intestinal mucosa and in particular of adenopolyposis coli, and of their progression to colorectal carcinoma.

### BACKGROUND OF THE INVENTION

Adenocarcinoma, or colorectal carcinoma (CRC), is the third cause of death in the world. CRC pathogenesis includes polyps formation onto the intestinal mucosa, considered pre-cancerous lesions. When diagnosed by colonoscopy, the intestinal polyps are usually endoscopically removed. After polypectomy, the recurrence rate is around 10-15 % per year. Among recurrent polyps, 10% occurs as advanced adenoma, characterised by a diameter ≥ 1 cm and histologically villous. The presence of one advanced adenoma, or the presence of two new formations after polypectomy, independently from their histological characteristics, characterise the population with the highest risk of developing a CRC (Vogelstein B et al. Genetic alterations during colorectal-tumor development. NEJM 1988, 319: 525-532).

Human familial adenomatous polyposis (FAP) is caused by a germinal mutation of the APC (Adenomatous Polyposis Coli) tumour suppressor gene. FAP is characterised by the development of a large number (hundreds to thousands) of adenomas in the colon. Whereas the genetic substrate favours the initiation of the tumorigenic process and cannot be pharmacologically or nutritionally modified, promotion of tumorigenesis may be controlled, so thwarting the progression toward cell de-differentiation and neoplastic transformation. The primary goal of the colonoscopic screening is the monitoring of adenomas and their progression to advanced forms and to CRC. In addition to colonoscopic screening, the clinical need for chemopreventive interventions, safe and effective in keeping low the recurrence rate of adenomas, is certainly high, but still unmet. Cyclooxigenase-2 (COX-2) inhibition by celecoxib and rofecoxib, although effective, is associated with an unbearable risk of cardio-vascular side effects (Lance P. Chemoprevention of colorectal cancer: some progress but a long way to go. Gastroenterology 2008, 134: 341-343*).*

A higher CRC prevalence in males, and a reduced incidence in females undergoing hormone replacement therapy (HRT) support the hypothesis that estrogens play a protective role in colon tumorigenic process. As a further proof of concept of the protective role of estrogens in the intestinal mucosa, it must be cited the recent evidence on the regression of the adenomas in a young woman, affected by FAP, after estro-progestinic therapy for contraceptive purposes *(*Giardiello F, Hylind L, Trimbath J et al. Oral Contraceptives and Polyp Regression in Familial Adenomatous Polyposis. Gastroenterology 2005; 128:1077-1080*).* Unfortunately, in the epidemiological studies conducted on post-menopausal women undergoing estro-progestinic therapy, a high incidence of breast cancer and cardiovascular accidents have been observed, thus requiring the early termination of the study to safeguard the health of the enrolled patients (Writing Group for the Women's Health Initiative Investigators. Risks and benefits of estrogen plus progestin in healthy postmenopausal women. JAMA 2002, 288: 321-333).

Estrogen receptors exist in two forms: alpha (ERα) and beta (ERβ), differently localised and expressed in the various anatomical districts. ERα is mainly localised in breasts, cardiovascular system, bones, urogenital and central nervous system, whereas ERβ is expressed in prostate, salivary glands, testes, ovaries, in the immune system and, predominantly, in the intestine. 17β-estradiol (estrogen) is a non selective ligand for both receptors, that performs a proliferative activity in presence of ERα and anti-proliferative in presence of ERβ (Koehler KF et al. Reflections on the discovery and significance of Estrogen Receptor β. Endocr Rev 2005, 26: 465-478). The beneficial effects observed in the above mentioned studies on the reduced CRC incidence could therefore depend specifically from the Erβ activation.

The involvement of ERβ in the adenomatous polyposis and CRC is demonstrated by its reduced expression, with respect to normal intestinal mucosa, in polypectomized patients already in pre-cancerous stage (Di Leo A et al. ER-β expression in large bowel adenomas: implications in colon carcinogenesis. Dig Liver Dis 2008, C40: 260-266). Significantly, ERβ reduction relates with an increased proliferation and a progressive cell de-differentiation, demonstrating that ERβ silencing is involved in tumour progression. The colorectal pre-cancerous lesions hence are early warnings of CRC development in a relevant part of the population. The dramatic decline of ERβ is also observed in the more advanced stages of the neoplastic transformation (Konstantinopoulos PA et al. Oestrogen receptor beta (ERβ) is abundantly expressed in normal colonic mucosa, but declines in colon adenocarcinoma paralleling the tumor's dedifferentiation. Eur J Cancer 2003, 39: 1251-1258*).*

Experimental studies conducted on C57BU6J - APC Min/+ transgenic mice, that carry a mutation of the germinal line of the APC gene, support the estrogens' protective role in the prevention of the neoplastic transformation in colonic epithelial cells. In particular, Weyant et al. (Weyant MJ et al. Reciprocal expression of ERα and ERβ is associated with estrogen-mediated modulation of intestinal tumorigenesis. Cancer Res 2001, 61: 2547-2551), using female transgenic mice, demonstrated that intestinal adenomas spontaneously develop after ovariectomy. Ovariectomy gave rise to a 77% increase in intestinal adenomas neoformation, whereas the subsequent administration of 17-β-estradiol caused a reduction in intestinal adenomas.

Besides genetic causes, it is known that exposure to carcinogens inside the intestinal lumen or to agents potentially genotoxic for the epithelial cells of intestinal mucosa is an epigenetic event that facilitates the inactivation of both alleles or the somatic mutation of the APC gene, both in FAP and sporadic form. These dangerous substances are normally ingested with the diet, or they can be formed in the intestinal lumen as an effect of the digestion, in particular of fats, or by degradation of bile acids.

For this reason, a diet rich in fibres, believed exerting a shielding effect on the intestinal mucosa or adsorbing potentially genotoxic xenobiotics, is considered useful in CRC prevention (Robertson DJ et al. Fat, fiber, meat and the risk of colorectal adenomas. Am J Gastroenterol 2005, 100: 2789-2795). However, the clinical evidences of such efficacy are not always convincing. This often depends on the poor quality of the various experimental protocols, where the expected effects are not weighed against the total fibre dietary intake, comprised of soluble and insoluble fibres. Therefore, the expected effects due to the addition to the diet of a known amount of a fibre qualified for its structure, degree of solubility and digestibility, with its own viscosity and gelling properties in the intestinal lumen, are largely unclear. All the previously mentioned features (i.e. structure, degree of solubility and digestibility, viscosity and gelling properties) heavily affect the properties of the fibre, as well as its biological effects on the intestinal physiology of complex organisms (Jones JR et al. Dietary reference intake: implication for fiber labeling and consumption: a summary of the international Life Sciences Institute North America Fiber workshop, June 1-2, 2004 Washington, DC. Nutr Rev 2006, 64k: 31-38).

Tamura M, et al. (J. Nutr. Sci Vit., 2003, 49, 168-171) disclose the effect of soy oligosaccharides on metabolic activity of intestinal microflora in mice fed with a soy oligosaccharide-isoflavone diet and cellulose-isoflavone diet, where isoflavones are a mixture of daidzein and genistein, whilst Stangroom K. et al. (Can. J. Phys. Pharm., 1984, 62, 1219-1224) disclose the effect in rats of a mixture of coumestrol in association with lignin on zearalenone toxicosis and DE 102004054133 describes the metabolic effect on plasma cholesterol, triglycerides and glucose of a mixture of silymarin, yacon and cellulose.

A putative effect of silibinin, as preventive agent, on colorectal cancer and colorectal liver metastasis has been supposed on the basis of detectable levels of silibinin in plasma and liver found in a clinical pilot study on twelve patients with colorectal carcinoma and twelve patients with colorectal liver metastasis orally treated with a composition of silibinin formulated with phosphatidylcholine (dosages: 360, 720 or 1440 mg/day for seven days) (Hoh C.S.I. et al., Proc. Am. Ass, Cancer Res. Annual Meeting, 2006, 47, 752*).*

The role of Bax in resveratrol-induced apoptosis of colorectal cancer has been assessed *in vitro* in human HCTI16 colon carcinoma cells, where both Bax alleles were inactivated (Mahyar-Roemer M. et al., BMC Cancer 2002, 2, 1471-2407) as well as and the role of genistein (*in vitro* and in doses ranged from 0.01 and 10 µM) in downregulatian of 25-D-3-1 in human mammary (MCF-7) and colonic (Caco-2) tumor cells (Lechner D. and Cross H., Proc. Am. Ass. Cancer Res. Annual Meeting, 2004, 45, 736*)* has been reported in literature.

The different metabolic effects of pectin, cellulose and lignin on stool pH, transit time and weight has been reported (Hillman L. et al., Brit. J. Nutr., 1983, 50, 189-196). On the basis of these findings the authors assumed that the analysis of the different types of dietary fibre components consumed may be relevant for understanding the colorectal cancer aetiology

In EP 0846704 a possible use of a partially degraded retrograded starch composition as food supplement for preventing colorectal digestive diseases, including colorectal cancer, is claimed on the basis of n-butyrate production under fermentation conditions simulating the human colon.

### SUMMARY OF THE INVENTION

According to the inventors, the therapeutic need may be met by locally delivering agents capable to act as selective agonists to the estrogen receptor-beta involved in the progression of the adenopolyposis to colorectal cancer, significantly reducing the systemic absorption or such agents and thus favouring their pharmacological action onto the intestinal mucosa.

Compositions for oral administration comprising an association of at least one phytoestrogen, selectively agonist to Estrogen Receptor Beta (ERβ), or more phytoestrogens with the above properties, and insoluble dietary fibres, i.e. fibres resistant to enzymatic digestion in the intestinal tract, may meet the need in the medical field of tools useful for prevention or treatment of the adenopolyposia coli condition and its progression to colorectal cancer.

Therefore, in a first aspect it is an object of the present invention compositions comprising at least one phytoestrogen or a mixture of phytoestrogens selectively agonists to the estrogen receptor-beta (ERβ), and at least one insoluble and indigestible fibre or mixtures thereof selected from the group consisting of non-starch polysaccharides.

Yet, it is a further object of the invention the use of such compositions orally administered for local treatment of the intestinal mucosa, and in particular the treatment of adenopolyposis coli conditions, and for the prevention of their progression to colorectal cancer.

As it will be readily apparent from the detailed description of the invention reported hereafter, in the presence of pre-cancer lesions of the colon characterised by mutations of the APC tumour suppressor, defined as polyps or adenomas, the compositions for oral administration can be profitably used to reduce the number and volume of polyps and to prevent their progression toward the neoplastic transformation, reducing their grade of dysplasia.

The compositions for oral administration object of the present invention can be profitably used to restore ERβ expression onto the colon mucosa, otherwise deficient or silenced when intestinal adenomas occur, and to restore a ratio of expression between the estrogen receptor beta and alpha in favour of the anti-proliferative activity.

Moreover, the association from at least one phytoestrogen, selective for the estrogen receptor-beta, and at least one insoluble dietary fibre forming the compositions for oral administration object of the present invention represents a preferential delivery system for the ERβ selective phytoestrogen to the colon, where the adenopolyposis is preferentially located, with an enrichment of the components onto the intestinal -mucosa and into the colonocytes. The oral compositions object of the present invention are therefor a safe and effective delivery system for local treatment of the intestinal mucosa, in conditions at high risk for the development of adenomas and their progression to adenocarcinoma, therefore useful in the treatment of subjects affected by adenopolyposis coli, in familial and sporadic forms, recruited to colonoscopic screening and after polypectomy, to prevent the development of advanced adenomas, and to prevent their potential neoplastic transformation into adenocarcinoma, also known as colorectal cancer (CRS).

### BRIEF DESCRIPTION OF THE FIGURES

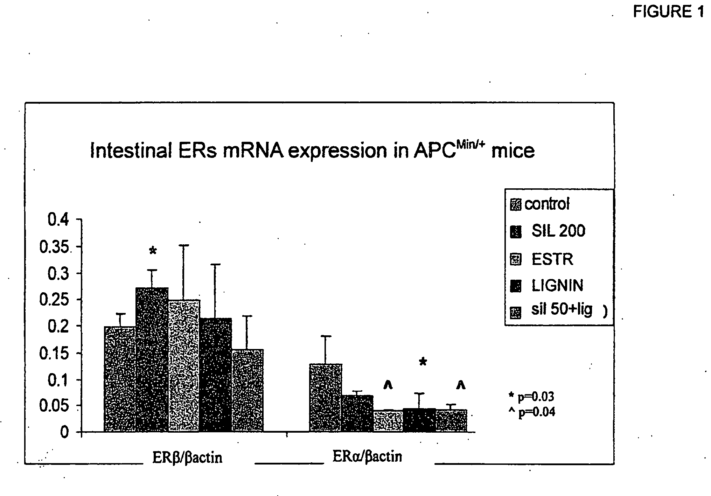

**Figure 1****.** The figure shows the genic expression (Messenger RNA, mRNA) of estrogen receptors in APC Min^{/+} mouse intestine. The levels of expression of ERβ and ERα mRNA are expressed as optical density units in comparison with the optical density units of constitutive 18S mRNA, relative to a structural protein unmodified in the experimental conditions of the present study. The results show: a significant increase of ERβ mRNA in the silymarin 200 ppm group (p=0.03), with respect to the control group fed standard diet; an unexpected significant decrease of the ERα mRNA in the group fed the insoluble fibre lignin alone (p = 0.04); a significant increase of ERβ in the group fed the composition formed by the association lignin+silymarin (p=0.04).

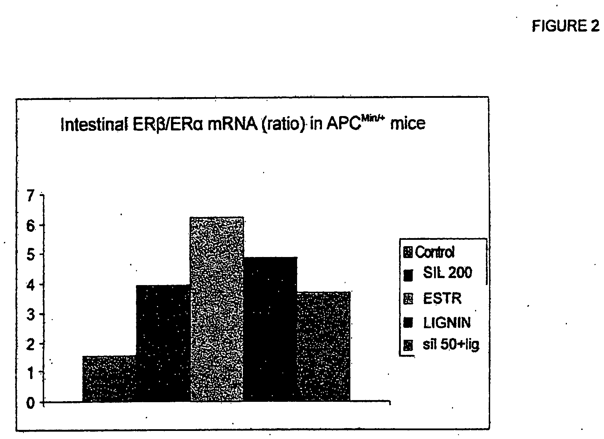

**Figure 2****.** The figure shows the ratio between the mRNAs of estrogen receptor alpha and beta, in the intestine of APC Min^{/+} mice. By comparison with the group of animals subcutaneously treated with estrogen (known to be non selectively active on ERβ and ERα), the results confirm the selectivity of silymarin for ERβ, being the receptor increased by the ligand. Moreover, the results demonstrate that the association of silymarin (even at low doses) with the insoluble fibre lignin, by itself surprisingly active in reducing the expression of ERα which possesses a proliferative activity, facilitates the restoration of an expression ratio between the estrogen receptors in favour of the anti-proliferative activity.

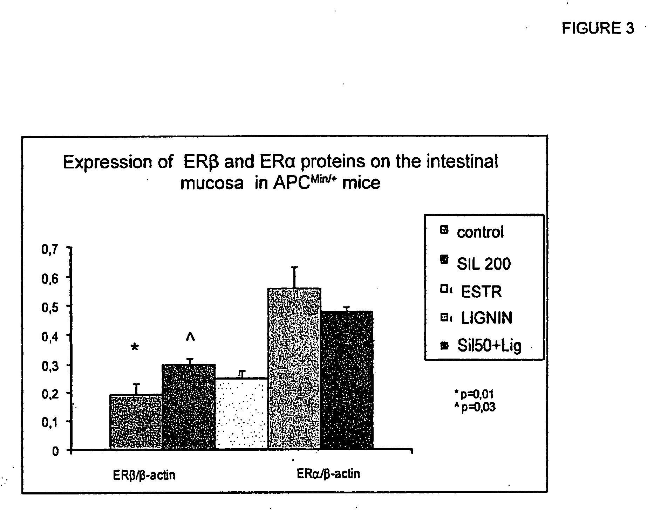

**Figure 3****.** The figure shows the ratio between the level of estrogen receptor alpha and beta proteins, in the intestine of APC Min^{/+} mice, as detected by Western Blotting. With respect to the control group of animals fed standard diet only, an increase of the ERβ protein versus the ERα protein is observed in animals fed different diets, this increase being higher in the group subcutaneously treated with 17β-estradiol; in the silymarin fed group; in the lignin fed group; in the group fed the composition containing lignin + silymarin, even at low doses.

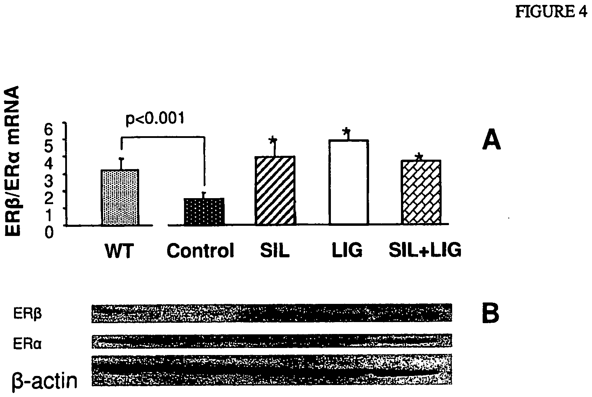

**Figure 4.** Figure 4A compares ERβ/ERα mRNA ratio in the small intestine of wild type (WT) and APC Min^{/+}groups, fed different diets. Silymarin (SIL), lignin (LIG) and silymarin+lignin (SIL+LIG) restored ERβ/ERα mRNA to levels comparable with WT healthy mucosa. ERβ protein was also increased in all the dietary managed groups (Fig. 4B), when compared to controls.

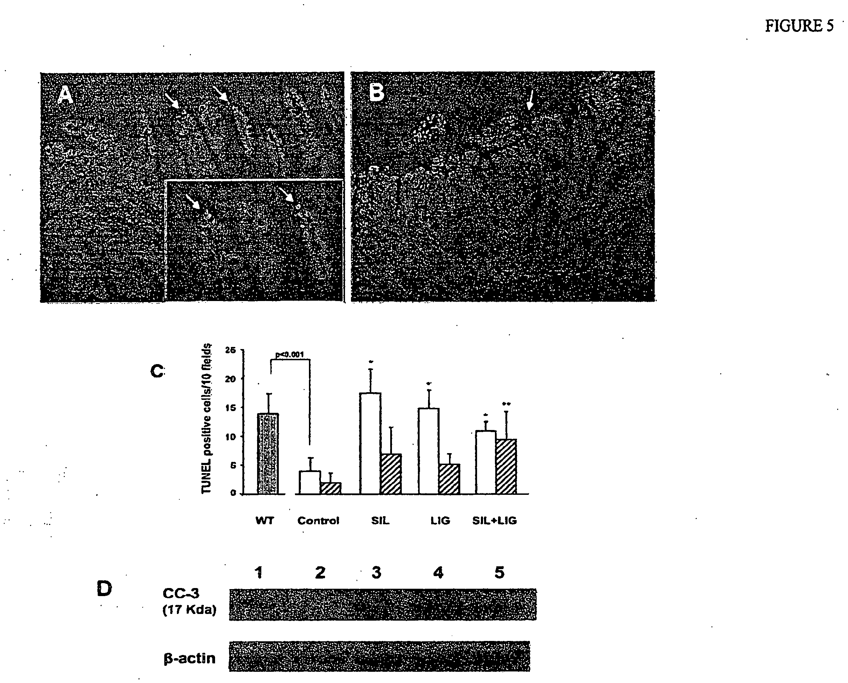

**Figure 5****.** TUNEL assay (A-C) and cleaved caspase-3 (CC-3) (D) assessment of pro-apoptotic acivity in non adenomatous and adenomatous mucosa from APC Min^{/+} animals fed the standard diet (Control) or different diets (SIL, LIG, SIL+LIG), in comparison with wild type (WT) mice fed the standard diet. In non-adenomatous mucosa, silymarin, lignin, and the combination of the two restored apoptosis to levels comparable with the healthy mucosa from WT mice. In adenomatous mucosa, only the association of silymarin and lignin restored apoptosis to values comparable with healthy mucosa from WT mice. Whereas CC-3 markedly decreased in the APC Min^{/+} mice fed the standard diet, a strikingly increase was observed in the dietary managed groups.

### DETAILED DESCRIPTION

According to the state of the art described above, and considering the side effects potentially experienced with non selective agonists of estrogen receptors, the oral compositions object of the present invention comprise at least one phytoestrogen selectively agonist to estrogen receptor beta (ERβ), or mixtures of such phytoestrogens, supported by a wide use that sustains its/their clear safety and effectiveness profile.

For the purpose of the present invention, the preferred phytoestrogens are selected from the group consisting of flavolignans, namely silymarin, silybinin, iso-silybinin, silydianin, silychristin, the isoflavone genistein, the flavonoid resveratrol, coumestrol and mixtures thereof. The phytoestrogen or phytoestrogens composing the mixture can be present in the composition both in their purified form and as plant extract. More preferably, the phytoestrogen is silymarin, as such or as a Milk Thistle extract, the most preferred one being the Milk Thistle extract consisting of a mixture of flavolignans silymarin, silybinin, iso-silybinin, silydianin and silychristin.

It is known that silymarin, a standardized mixture of flavolignans extracted from Milk Thistle fruits, widely used in traditional medicine as hepatoprotectant, possesses a documented profile of safety and tolerability, as well as properties of the same as selective ERβ agonist, without any relevant activity on Erα, have been described (Seidlova-Wuttke D et al. Silymarin is a selective estrogen receptor β (ERβ) agonist and has estrogenic effect in the metaphysis of the femur but no or antiestrogenic effects in the uterus of ovariectomized (ovx) rats. J Steroid Biochem Mol Biol 2003, 86: 179-188).

"Silymarin" is the common denomination used for a plant extract containing a mixture of flavolignans: silymarin, silybinin, iso-silybinin, silydianin and silychristin in variable concentrations and ratios. In the compositions object of the present invention, "Silymarin" means the mixture of flavolignans composing the extract, in different concentrations and ratios, with particular, reference to a Milk Thistle extract with an analytical profile characterised by a silymarin content higher than 70% and a silybinin A+B content higher than 30%. In general, additional components to and/or substitutes of silymarin in the compositions object of the present invention are other phytoestrogens, provided that they are selective for ERβ and with a anti-estrogenic activity, that is not inducing a significant proliferative activity on tissues that are classical targets for the estrogens, such the above mentioned phytoestrogens, in similar concentrations as that of silymarin.

The other essential component of the compositions for oral administration of the present invention is an insoluble and indigestible, i.e. resistant to enzymatic digestion in the gastrointestinal tract, fibre. The insoluble fibre can be present in a purified form or as component of a natural' extract, provided that in the compositions the insoluble fibre content is between 1% and 30%. Such fibre is selected from plant fibres with a non-starch polysaccharidic nature and among these fibres are preferably fibres selected in the group consisting of polyphenylpropanoid lignin (hereafter called also lignin), cellulose, inulin and long chain inulin and mixtures thereof. Particular preference is given to the lignin. The insoluble fibre can be present as a purified component as well as a constituent of an extract. In the later case, the above mentioned insoluble fibres can be present as a mixture and associated with other nutritional substances. In particular, the fibres can be used for the compositions of the invention as follows: in the case of lignin, as such In the polydisperse form or as wheat bran; in the case of cellulose, as such or as a soy extract; in the case of inulin, long chain inulin is preferable, with a polymerisation degree of 10-65 and an average chain length of 25, as such or as artichoke extract, chicory extract or Dandelion root powder.

The fibres present in the oral compositions object of the present invention are anyway to be considered add-ons to the diet, but independent from the total dietary fibre intake.

The compositions according to the inventions have shown unexpected biological properties, since said compositions, characterized substantially by the association from at least one phytoestrogen, selectively agonist to estrogen receptor beta (ERβ), or mixtures thereof and at least one dietary insoluble fibre selected from fibres having a non-starch polysaccharidic nature or mixtures thereof, proved to have a synergistic effect on the parameters used for the necessary experimental validation of their usefulness.

In fact, the dietary insoluble fibre (in particular lignin) proved to be active in maintaining ERβ proteic expression, evidence unknown to date, and proved to be synergic with the phytoestrogen silymarin in significantly keeping under control parameters that are markers of the proliferation and neoplastic progression, such as for example the volume and degree of dysplasia of adenomatous polyps.

In addition similar synergistic unexpected effects between phytoestrogens and fibres have been seen in other relevant biological parameters, such as the restoration of a pro-apoptotic activity on the adenomatous mucosa and even more interestingly on the non-adenomatous mucosa, to level comparable with those observed on healthy intestinal mucosa. The unbalanced proliferation/apoptosis ratio observed on the non adenomatous intestinal mucosa of APC Min^{/+} mice fed the standard diet - an experimental model validated to assess both the pathobiological mechanisms as well as the efficacy of pharmaceutical and/or nutraceutical interventions of human clinical relevance - can explain the clinically documented recurrence of polyps in patients entered the screening colonoscopy program, because of previous polyp detection and polypectomy, and regarded as at increased risk of CRC development and progression.

The results obtained above mentioned in summary widely support the envisaged use of the compositions object of the present invention. In particular, in mammals, humans or non-humans, a composition for oral administration according to the invention can induce the expression of the estrogen receptor beta directly on the intestinal mucosa, when such receptor is poorly expressed or absent because of the presence of pre-cancerous lesions in the colon mucosa, due to a mutation of the APC tumoral suppressor, such as per familial (FAP) and sporadic adenopolyposis.

In addition, the oral compositions according to the present invention could be a useful system to preferentially target the delivery of the phytoestrogens to the intestinal area affected from polyp development, being this area the small intestine in the experimental mouse model and the colon in humans, where the insoluble fibres are partially digested. The insoluble fibres contained in the compositions according to the present invention, and particularly lignin, act as a shield in the upper gastrointestinal tract, where phytoestrogens are otherwise preferentially absorbed. Moreover, this shielding effect allows a prolonged contact of the phytoestrogens comprised in the compositions according to this invention onto the intestinal mucosa and onto colonocytes. As a proof of concept, whereas the insoluble fibre, and particularly lignin, was *per* se ineffective in counterfighting intestinal neoplasia, as assessed by the number of developed polyps and their volume, the compositions according to the invention, and particularly the combination of silymarin at the lowest effective dose with lignin determined a maximal reduction in polyp number and volume, comparable with the one obtained by adding the standard diet with silymarin at the highest effective dose.

Compositions according to the invention can therefore usefully be employed, either as pharmaceutical products or nutraceuticals, in the treatment of patients affected by colon adenomas, diagnosed through colonoscopic examination, with the aim of reducing polyp number and volume, of preventing their progression to advanced adenomas and, possibly, to adenocarcinoma. Compositions according to the invention can also be employed to prevent or reduce the development of new polyps and to prevent their progression to an advanced form, and possibly to adenocarcinoma, in polypectomised patients currently recruited to screening colonoscopy program, in the period between scheduled screening colonoscopies. The compositions for oral administration of the present invention can be in both liquid and solid form. Independently from the type of phytoestrogens used and from the form thereof (i.e in purified form or as extract), in said compositions the phytoestrogens and the mixtures thereof are comprised from 0.1% to 10% by weight, more preferably from 0.5% to 5%, and most preferably from 1% to 2%.

In fact, when the phytoestrogen is silymarin, said solid and liquid oral compositions preferably comprise from 0.1% to 10% by weight, more preferably from 0.5% to 5%, and most preferably from 1% to 2% of silymarin, independently from the extract thereof used.

In the case of use of genistein as phytoestrogen, the solid and liquid compositions of the present invention preferably comprise from 0.1% to 10% by weight, more preferably from 0.5% to 5%, and most preferably from 1% to 2% of genistein, independently from the extract used.

The solid and liquid compositions object of the present invention preferably comprise from 0.1% to 10% by weight, more preferably from 0.5% to 5%, and most preferably from 1% to 2% of coumestrol, independently from the extract used.

The solid and liquid compositions object of the present invention preferably comprise from 0.1% to 10% by weight, more preferably from 0.5% to 5%, and most preferably from 1% to 2% of mixed phytoestrogens, independently from the extract used.

With reference to the insoluble fibre component, the solid compositions of the present invention preferably comprise from 1% to 30% by weight, more preferably from 5% to 25%, and most preferably from 10% to 20% of indigestible dietary fibre, independently from the type of extract used (i.e. purified or as such).

The liquid compositions of the present invention preferably comprise from 1% to 20%, more preferably from 2% to 15%, and most preferably from 5% to 10% of indigestible and insoluble dietary fibre, independently from the type of extract used (i.e. purified or as such).

The solid compositions can be in form of powder, effervescent powder or chewable tablets. The powder forms can in turn be multi-dose (bottles, canisters, jars) and single dose (sachets) packages. The liquid forms can be in form of syrups, solutions, suspensions or aqueous dispersions. In such formulations, the compositions can additionally comprise acceptable pharmaceutical or food grade excipients, diluents, preservatives, flavours, foaming agents, pH correcting agents, etc., depending on the chosen form, suitable for the oral administration.

Moreover, the compositions of the present invention can further comprise probiotics, such as for example lactobacilli and/or bifidobacteria, both live and tyndalized, with the purpose of contributing to the normalization of the intestinal microflora and to the intestinal fermentation of the fibre, effects known contributing to the formation of a healthy microenvironment in this anatomical district. Probiotics are added as adjuvants to maintain or balance the intestinal physiology and to help metabolise the indigestible and insoluble fibre.

Compositions in accordance with the present invention are shown in working examples 1-10. Working examples 1-7 refer to solid formulations, and working examples 8-10 to liquid formulations. Unless otherwise specified, the proportions of the components comprising the compositions in the working examples represent the weight percentages of the respective components relative to the final product. The following examples further describe and demonstrate embodiments within the scope of the present invention. The examples are given solely for the purpose of illustrations, and are not intended to limit the scope of the present invention, because variations of the present invention are possible without departing from the spirit and scope of the invention.

### Solid formulations

### Example 1: Effervescent powder

| **Component** | **Quantity %** |
|---|---|
| Milk Thistle Dry Extract 70% HPLC silymarin | 2.00 |
| Corresponding to silymarin | 1.40 |
| Lignin | 20.00 |
| Maltodextrins | 70.90 |
| Sodium Citrate | 4.50 |
| Potassium Citrate | 0.50 |
| Citric Acid | 1.00 |
| Silicon Dioxide | 0.10 |
| Flavouring | 1.00 |

### Example 2: Effervescent powder

| **Component** | **Quantity %** |
|---|---|
| Milk Thistle Dry Extract 70% HPLC silymarin | 2.00 |
| Corresponding to silymarin | 1.40 |
| Oligosaccharide Fibre (Inulin) | 20.00 |
| Maltodextrins | 70.90 |
| Sodium Citrate | 4.50 |
| Potassium Citrate | 0.50 |
| Citric Acid | 1.00 |
| Silicon Dioxide | 0.10 |
| Flavouring | 1.00 |

### Example 3: Effervescent powder

| **Component** | **Quantity %** |
|---|---|
| Milk Thistle Dry Extract 70% HPLC silymarin | 2.00 |
| Corresponding to silymarin | 1.40 |
| Lignin or Long Chain Inulin | 20.00 |
| Lactobacillus Acidophilus, Tyndalised | Equivalent to 10 MLD |
| Maltodextrins | 71.90 |
| Sodium Bicarbonate | 2.60 |
| Tartaric Acid | 1.40 |
| Silicon Dioxide | 0.10 |
| Flavouring | 2.00 |

### Example 4: water-dispersible powder

| **Component** | **Quantity %** |
|---|---|
| Silymarin | 1.40 |
| Dandelion Root Powder | 35.00 |
| Maltodextrins | 61.50 |
| Silicon Dioxide | 0.10 |
| Flavouring | 2.00 |

### Example 5: Effervescent powder

| **Component** | **Quantity %** |
|---|---|
| Milk Thistle Dry Extract 70% HPLC silymarin | 1.00 |
| Corresponding to silymarin | 0.70 |
| Genistein | 0.70 |
| Dietary or Oligosaccharidic Fibre (Inulin) | 20.00 |
| Maltodextrins | 70.90 |
| Sodium Citrate | 4.50 |
| Potassium Citrate | 0.50 |
| Citric Acid | 1.00 |
| Silicon Dioxide | 0.10 |
| Flavouring | 1.00 |

### Example 6: Effervescent powder

| **Component** | **Quantity %** |
|---|---|
| AlfaAlfa Dry Extract 10% HPLC coumestrol | 7.00 |
| Corresponding to coumestrol | 0.70 |
| Wheat Bran | 30.00 |
| Maltodextrins | 55.90 |
| Sodium Citrate | 4.50 |
| Potassium Citrate | 0.50 |
| Citric Acid | 1.00 |
| Silicon Dioxide | 0.10 |
| Flavouring | 1.00 |

### Example 7: water-dispersible powder

| **Component** | **Quantity %** |
|---|---|
| Silymarin | 0.70 |
| Genistein | 0.70 |
| Dandelion Root Powder | 35.00 |
| Maltodextrins | 61.50 |
| Silicon Dioxide | 0.10 |

### Liquid formulations

### Example 8: Syrup

| | **Quantity in 100 ml** |
|---|---|
| **Component** | **Grams** |
| Silymarin | 0.90 |
| Lignin, Cellulose or Inulin | 10.00 |
| Potassium Sorbate | 0.10 |
| Ascorbic Acid | 0.50 |
| Citric Acid, Anhydrous | 0.05 |
| Fructose | 35.00 |
| Flavouring | 0.35 |
| Water | Q.S. |

### Example 9: Syrup

| | **Quantity in 100 ml** |
|---|---|
| **Component** | **Grams** |
| Silymarin | 0.45 |
| Genistein | 0.45 |
| Lignin, Cellulose or Inulin | 10.00 |
| Potassium Sorbate | 0.10 |
| Ascorbic Acid | 0.50 |
| Citric Acid, Anhydrous | 0.05 |
| Fructose | 35.00 |
| Flavouring | 0.35 |
| Water | Q.S. |

### Example 10: Syrup

| | **Quantity in 100 ml** |
|---|---|
| **Component** | **Grams** |
| Milk Thistle Dry Extract 70% HPLC silymarin | 1.38 |
| Corresponding to silymarin | 0.90 |
| Dandelion Root Powder | 20.00 |
| Potassium Sorbate | 0.10 |
| Sodium Benzoate | 1.0 |
| Ascorbic Acid | 0.5 |
| Citric Acid | 0.15 |
| Sorbitol | 20.00 |
| Saccharin | 0.013 |
| Flavouring | 0.20 |
| Purified Water | Q.S. |

### Experimental part

The C57BL/6J APC Min/+ transgenic mouse is an excellent animal model for FAP, because the mutated APC gene is homologous to the human one. Even in the most common form of CRC, called sporadic, 85% of cases show the somatic mutation of the APC tumour suppressor gene. Therefore, the APC mutated transgenic animal model is acknowledged as the most significant for the study of the pathogenetic mechanisms that characterise the adenomatous polyposis in the colon, both in FAP and sporadic form, and it is considered a validated animal model for the set up of nutritional and pharmacological interventions aimed at controlling the development of adenomas and at preventing their progression to CRC (Levy DB et al. Inactivation of both APC alleles in human and mouse tumors. Cancer Res 1994, 54: 5953-5958*; Paulsen JE et al. Qualitative and quantitative relationship between dysplastic aberrant crypt foci and tumorigenesis in the Min*/*+ mouse colon*). For this reason, in setting up the oral formulations object of the present invention, and in the evaluation of their safety and efficacy, the Inventors made use of the above mentioned C57BL/6J APC Min/+ transgenic mouse model. Hereafter are described the data showing the effect in C57BL/6J - APC Min/+ transgenic mice, orally fed silymarin added diets, containing soluble and insoluble fibres in different proportions with the purpose of highlighting the differentiative properties of the insoluble and indigestible fibre.

The three diets under examination, formulated with the aim of mimicking a fat rich dietary regimen, were as follows:
- Diet 5K20 [Mucedola Srl, Settimo Milanese (MI)], containing 4.50% of total fibre, composed of soluble and insoluble fibres;
- Purified diet containing 6.24% of insoluble fibre, constituted by lignin;
- Purified diet containing 6.24% of soluble fibre, constituted by Plantago ovata husk.

Silymarin up to 200 mg/kg was added to each diet. The diets were orally administered for 10 weeks to 114 male C57BL/6J - APC Min/+ transgenic mice 5-6 weeks old. These mice have a mutation of the APC gene, and are prone to develop colon adenomatous polyposis. Objective of the study was to verify the capacity of the composition according to the present invention in significantly reducing polyps development and preventing their progression to severely dysplastic forms, markers of neoplastic transformation, in an animal model recognised as comparable to the human pathologic situation. The statistical analysis has been performed by means of the One-way Analysis of Variance and the t-Student tests, considering statistically significant a p<0.05.

The animals were divided into 14 groups, as reported in Table 1. Every oral treatment lasted 10 weeks. The treatment scheme was designed to highlight the effects of the type of fibre, associated or not with increasing amounts of silymarin. As a positive control, a group of transgenic mice fed standard diet (5K20, Mucedola) and subcutaneously implanted with a pellet containing 1.5 mg of 17β-estradiol (estrogen) was used.

**Table1. Number of animals per group (sil= silymarin, ppm=part per million)**

| | Standard diet | Diet with Plantago ovata husk | Diet with lignin |
|---|---|---|---|
| + sil 0 ppm | 15 | 10 | 10 |
| + sil 10 ppm | 6 | 6 | 6 |
| + sil 50 ppm | 6 | 6 | 6 |
| + sil 200 ppm | 15 | 6 | 6 |
| + sil 600 ppm | 7 | | |
| + estrogen (s.c., control) | 9 | | |

No difference in the daily dietary intake (about 3±0.1 g/day) was reported among animals in the different groups. During the treatment period, the mortality rate was low in the groups fed standard diet or diet added with compositions described in this patent application (7.1%), whereas in groups fed diet added with Plantago ovata husk the mortality was very high (>50%).

Table 2 list the means and standard deviations of the weight of the animals from different diet groups, at time of sacrifice. At admission to the animal house their average weigh was 19 ±1.2 g.

**Table 2. Mean and standard deviation of body weights at time of sacrifice**

| | Standard diet | Diet with lignin |
|---|---|---|
| + sil 0 ppm | 22.0 ± 3.8 g | 25.0± 1.9g |
| + sil 10 ppm | 26.0 ± 1.9 g | 25.2 ± 2.3 g |
| + sil 50 ppm | 24.6 ± 3.3 g | 26.9 ± 2.1 g |
| + sil 200 ppm | 23.5 ± 2.6 g | 27.9 ± 1.7 g |
| + sil 600 ppm | 24.3 ± 2.4 g | - |
| + estrogen (s.c., control) | 24.9 ± 2.8 g | |

Differently from animal groups fed the diet containing P. ovata husk soluble fibre, in which a high mortality rate and no reduction in polyp numbers were observed, the association of silymarin and lignin constituting one of the compositions according to the present invention proved to be safe and well tolerated, and resulted highly effective in keeping down the number, volume, and grade of dysplasia of adenomatous polyps in the C57BL/6J - APC Min/+ transgenic mouse model. In addition, the dietary insoluble fibre (in this case lignin) proved to be active in maintaining ERβ proteic expression, evidence unknown to date, and proved to be synergic with silymarin in significantly keeping under control parameters that are markers of the proliferation and neoplastic progression, such as for example the volume and degree of dysplasia of adenomatous polyps.

After 10 weeks of treatment the animals received an intraperitoneal injection of 0.75 mg of Bromodeoxyuridine (BrdU), equivalent to 30 mg/kg, to verify the migration of cells of the intestinal mucosa along the villus axis (from crypt at the bottom to the luminal tip). This parameter allows to verify the normal esfoliative physiology of the intestinal mucosa, through which the mucosa keeps itself composed of healthy and vital cells.

To this end, the animals were sacrificed 24, 48 and 72 hours after injection. Small intestine and colon were taken from every sacrificed animal. The small intestine was divided into three sections: proximal, medial and distal. The proximal tract was further divided in two portions that were subsequently scraped. One of the two was dipped in RNAlater^{®} and stored at -20°C and the other stored in liquid nitrogen. The two samples were used for Real Time PCR and Western Blotting, respectively. The medial and distal tracts, together with the colon, were used for the histological analysis. After a 3-hours pre-fixative treatment with formol, the intestinal tracts under examination were observed under an STM1 stereomicroscope, 3x magnification. Two blinded observers measured the number and size of polyps.

Table 3 lists mean and standard deviation (SD) of the number of polyps observed in the animals fed the different diets. It has to be noticed that silymarin added at 200 ppm to the standard diet significantly reduces the number of polyps (p=0.005), at a level comparable with the reduction observed in the transgenic mice of the control group, subcutaneously treated with estradiol. Lignin alone is ineffective on this parameter, but the association of lignin and silymarin, object of the present invention, significantly reduces the number of polyps, at a silymarin dose (50 ppm) four times lower than that required to obtain the same results with standard diet (200 ppm).

**Table 3. Number of intestinal polyps in animals fed different diets**

| | Standard diet | Diet with Plantago | Diet with lignin |
|---|---|---|---|
| + sil 0 ppm | 64.8 ± 20.2 | 43.6 ± 9.5 | 46.8 ± 14.0 |
| + sil 10 ppm | 44.0 ± 21.8 | 46.4 ± 23.8 | 43.8 ± 19.0 |
| + sil 50 ppm | 55.4 ± 26.2 | 51.5 ± 20.5 | 34.0 ± 10.7* |
| + sil 200 ppm | 57.2 ± 9.0* | 50.0 ± 4.7 | 48.5 ± 2.5 |
| + sil 600 ppm | 52.6 ± 24.6 | | |
| Estrogen (control) | 32.33 ± 12.06* | | |

Table 4 lists the silymarin content (expressed as µg/g silybinin in the tissue, determined by HPLC) in the duodenal tract, where silymarin is known to be preferentially absorbed, and in the ileum after seven days feeding 200 ppm silymarin, or a composition containing 200 ppm silymarin and a non starch polysaccharidic fibre (lignin) as an add-on to the standard diet of C57BL/6J mice (n=6). The association of silymarin and insoluble fibre caused a preferential transport of the phytoestrogen in the ileal region, the intestinal area where polyps tipically develop in the transgenic mouse. Moreover, it is known that such condition mimics the development of adenopolyposis in human colon. Therefore, the compositions object of the present invention can be an effective system for the espace treatment of the area affected by adenomatous formations.

**Table 4. Mean and standard deviation of silymarin tissue concentration in mouse intestine**

| | Duodenum (µg/g) | lieum (µg/g) |
|---|---|---|
| Silymarin 200 ppm, 7 days | 6.54 ± 2.34 | 2.84 ± 2.03 |
| Silymarin 200 ppm + Lignin, 7 days | 2.47 ± 2.38 | 3.13 ± 2.71 |

Table 5 lists mean and standard deviation (SD) of the volume of intestinal polyps. Assuming polyps shape as a sphere, by measuring their diameter it was possible to calculate their volume, expressed in mm³. Surprisingly, lignin alone is able to reduce polyps volume (p=0.0001). The association of silymarin with lignin is able to significantly reduce the volume of polyps (p=0.0001). Moreover, the association is synergic for the two components, as suggested by the fact that the using lower doses of phytoestrogen, the same effect as per the higher doses of single component is observed. Noteworthy, estradiol proved to be ineffective on this parameter, demonstrating that the composition object of the present invention, containing a phytoestrogen selectively agonist to ERβ, is effective on this parameter.

**Table 5. Comparison of polyps volume (mm³ ± SD) in animal fed the different diets**

| | Standard diet | Diet with lignin |
|---|---|---|
| + sil 0 ppm | 242,1 ± 105.1 | 92.3 ± 81.8* |
| + sil 10 ppm | 145.0 ± 100.6 | 121.0 ± 72.6 |
| + sil 50 ppm | 158.4 ± 60.3 | 99.00 ± 53.13* |
| + sil 200 ppm | 120.2 ± 69.0* | 138.0 ± 63.3 |
| + sil 600 ppm | 162.07 ± 89.35 | |
| Estrogen (control) | 141.78 ± 79.1 | |

Histological preparations of randomly chosen intestine sections of every animal from the different treatment groups were observed by a blinded pathologist, who graded the dysplasia of the adenomatous formations present as minor, mild or severe. In the estradiol-treated animals, 70% showed polyps with dysplasia classifiable as severe, versus 21.4% in animals fed standard diet added with 200 ppm silymarin, 30% in animals fed diet with lignin, and 20% in animals fed diet with lignin added with 50 ppm silymarin.

Table 6 lists the results concerning HLC (highest labeled cell) In the experimental groups. Data shows that cell migration rate, and consequently the esfoliation rate of enterocytes in the intestinal lumen, is significantly increased in the group treated with lignin and silymarin (even at low doses).

**Table 6. Percentage of colonocytes migration from the crypt to the apex of the villus in transgenic animals of the different groups**

| | 24 hours migration (%) |
|---|---|
| Standard diet | 28 ± 2.6 |
| + estrogen | 47.3 ± 10.5* |
| + sil 200 ppm | 46.6 ± 7.0* |
| + lignin | 35 ± 13.8 |
| + (Lignin + sil 50 ppm) | 44.4 ± 11.8* |

With regard to the mechanism of action of the association silymarin and lignin, data demonstrates for the first time a potentiation of the ERβ expression on the intestinal mucosa. RT-PCR results are shown in Figure 1. The levels of expression of messenger RNA for ERβ and ERα are reported as average ratio between the optical density of the messenger for each receptor and that of 18S rRNA, relative to β-actin, a structural protein that is unmodified in the experimental conditions of the study.

The results show a significant increase of ERβ mRNA for in the silymarin 200 ppm group (p=0.03), with respect to the group fed standard diet.

Surprisingly, the Inventors demonstrated for the first time that the ERα mRNA is significantly decreased in the group treated with the insoluble fibre (p=0.04); the reduction is more pronounced in the group fed with a composition containing the association silymarin-lignin (p=0.04). From comparison with the group subcutaneously treated with estrogen (active on both ERβ and ERα), data confirms silymarin as selective ligand to ERβ and demonstrates that the association of silymarin (even at low doses) and lignin - the latter surprisingly active in reducing expression of ERα known to exert proliferative properties - further facilitates the re-establishment of a ratio between the two estrogen receptors in favour of the antiproliferative activity, as shown in Figure 2 and demonstrated by the data in Tables 3-6.

Figure 3 shows the level of intestinal expression of the estrogen receptors, as ratio between ERβ and ERα proteins, determined using the Western Blotting method. Compared to the group of animals fed standard diet only, an increase of ERβ as a protein in comparison to ERα protein was observed, and this increase is more marked in the group subcutaneously treated with 17β-estradiol, thus confirming the protective role hypothesised for the estrogens; in the silymarin-treated group, thus confirming its properties as a phytoestrogen selective to ERβ; and, surprisingly, in both groups treated with lignin and with lignin + silymarin, even at low doses.

Additional experimental were collected in order to provide evidence of: A) altered proliferative and apoptotic activities even on the non adenomatous mucosa of APC Min/+ animals, in comparison with syngeneic wild type (WT), no APC mutated, C57BL/6J intestinal mucosa (regarded as normal and healthy), being both animal groups fed the standard diet; B) the capability of the oral compositions object of the present invention to restore ERβ expression, proliferative and apoptotic activity in APC Min/+ mice, to levels comparable to the ones observed onto the normal mucosa of WT animals fed the standard diet. Therefore, these evidences further support the use of such oral compositions as an useful adjunct to screening colonoscopy program in previously polipectomised patients, at increased risk of developing new polyps (recurrence). Figure 4 (A, B) shows the ratio between ERβ and ERα mRNAs and the level of expression for both receptors as a protein, onto the non-adenomatous mucosa of WT and control APC Min/+ fed the standard diet, or APC Min/+ fed different diets. Figure 5 (A-D) demonstrates that: i) the non adenomatous mucosa of APC Min/+ mice fed the standard diet has lost pro-apoptotic control on cell proliferation when compared to the WT mice fed the standard diet, as detected by markers of apoptosis, such as the reduced number of positive cells on the TUNEL assay and cleaved capsase 3 protein expression, ii) the oral compositions object of the present invention, with particular reference to the association of silymarin and lignin, restore apoptotic control on cell proliferation to levels similar with the ones observed onto the normal, healthy intestinal mucosa of WT mice.

## Claims

1. A composition comprising at least one phytoestrogen selectively agonist to the estrogen receptor beta and at least one insoluble and indigestible fibre or mixtures thereof selected from the group consisting of non-starch polysaccharides for use in the prophylactic or therapeutic local treatment of intestinal mucosa pathological conditions selected from the group consisting of adenopolyposis and its progression to advanced adenomas and adenocarcinoma by oral administration.

2. The composition according to claim 1, wherein the phytoestrogens, in purified form or as extracts, are selected from the group consisting of silymarin, silybinin, iso-silybinin, silydianin, silychristin, genistein, resveratrol, coumestrol and mixtures thereof.

3. The composition according to claim 1, wherein the phytoestrogen is the Milk Thistle extract silymarin consisting of the mixture of silymarin, silybinin, iso-silybinin, silydianin, silychristin.

4. The composition according to claim 1, wherein insoluble :and indigestible fibres, in purified form or as extracts, are selected from the group consisting of lignin, cellulose, inulin, long chain inulin and mixtures thereof.

5. The composition according to claims 2-4, wherein the phytoestrogen is silymarin and the insoluble and indigestible fibre is lignin.

6. The composition according to claims 1-5, wherein phytoestrogens are in a range comprised from 0.1 to 10 % of the total weight of said composition.

7. The composition according to claims 6, wherein phytoestrogens are in a range comprised from 0.5 to 5% of the total weight of said composition.

8. The composition according to claims 6, wherein phytoestrogens are in a range comprised from 1 to 2% of the total weight of said composition.

9. The composition according to claims 1-5, wherein the insoluble and indigestible fibres are in a range comprised from 1 to 30% of the total weight of said composition.

10. The composition according to claim 9, wherein the fibres are in a range comprised from 5 to 25% in solid formulations.

11. The composition according to claim 9, wherein the fibres are in a range comprised from 10 to 20% in solid forms.

12. The composition according to claim 9, wherein the fibres are in a range comprised from 2 to 15% in liquid forms.

13. The composition according to claim 9, wherein the fibres are in a range comprised from 5 to 10% in liquid forms.

14. The composition according to any one of the preceding claims 1-13, wherein probiotics, live or tyndalized, are further added.

15. The composition according to any one of the preceding claims 1-14, in combination with pharmaceutically acceptable excipients and/or diluents suitable for solid forms selected from the group consisting of powders, effervescent powders, chewable tablets and tablets.

16. The composition according to any one of the preceding claims 1-14, in combination with food grade acceptable excipients and/or diluents suitable for solid forms selected from the group consisting of powders, effervescent powders, chewable tablets and tablets.

17. The composition according to any one of the preceding claims 1-14, in combination with pharmaceutically acceptable excipients and/or diluents suitable for liquid forms selected from the group consisting of syrups, solutions, suspensions and aqueous dispersions.

18. The composition according to any one of the preceding claims 1-14, in combination with food grade acceptable excipients and/or diluents suitable for liquid forms selected from the group consisting of syrups, solutions, suspensions and aqueous dispersions.

19. Use of at least one phytoestrogen selectively agonists to the estrogen receptor-beta and at least one insoluble and indigestible fibre or mixtures thereof selected from the group consisting of non-starch polysaccharides for preparation of the compositions according to any one of the preceding claims for the prophylactic or therapeutic local treatment of intestinal mucosa pathological conditions selected from the group consisting of adenopolyposis and its progression to advanced adenomas and adenocarcinoma by oral administration.

## Patentansprüche

1. Zusammensetzung umfassend mindestens einen Phytoöstrogen selektiven Agonisten für den Östrogenrezeptor Beta und mindestens eine unlösliche und unverdauliche Faser oder Mischungen daraus, ausgewählt aus der Gruppe bestehend aus Nicht-Stärke-Polysacchariden zur Verwendung für die prophylaktische oder therapeutische lokale Behandlung von pathologischen Zuständen der Darmschleimhaut, ausgewählt aus der Gruppe bestehend aus adenomatöser Polyposis und deren Progression zu fortgeschrittenen Adenomen und Adenokarzinom mittels oraler Verabreichung.

2. Zusammensetzung gemäß Anspruch 1, wobei die Phytoöstrogene, in gereinigter Form oder als Extrakte, aus der Gruppe ausgewählt sind, bestehend aus Silymarin, Silybinin, Isosilybinin, Silydianin, Silychristin, Genistein, Resveratrol, Coumestrol und Mischungen daraus.

3. Zusammensetzung gemäß Anspruch 1, wobei es sich bei dem Phytoöstrogen um aus Mariendistel extrahiertes Silymarin handelt, bestehend aus der Mischung von Silymarin, Silybinin, Isosilybinin, Silydianin, Silychristin.

4. Zusammensetzung gemäß Anspruch 1, wobei die unlöslichen und unverdaulichen Fasern, in gereinigter Form oder als Extrakte, aus der Gruppe ausgewählt sind, bestehend aus Lignin, Zellulose, Inulin, langkettigem Inulin und Mischungen daraus.

5. Zusammensetzung gemäß Ansprüchen 2-4, wobei es sich bei dem Phytoöstrogen um Silymrin handelt und die unlösliche und unverdauliche Faser Lignin ist.

6. Zusammensetzung gemäß Ansprüchen 1-5, wobei die Phytoöstrogene in einem Bereich vorliegen, umfassend 0,1 bis 10 % des Gesamtgewichts an genannter Zusammensetzung.

7. Zusammensetzung gemäß Anspruch 6, wobei die Phytoöstrogene in einem Bereich vorliegen, umfassend 0,5 bis 5 % des Gesamtgewichts an genannter Zusammensetzung.

8. Zusammensetzung gemäß Anspruch 6, wobei die Phytoöstrogene in einem Bereich vorliegen, umfassend 1 bis 2 % des Gesamtgewichts an genannter Zusammensetzung.

9. Zusammensetzung gemäß Ansprüchen 1-5, wobei die unlöslichen und unverdaulichen Fasern in einem Bereich vorliegen, umfassend 1 bis 30 % des Gesamtgewichts an genannter Zusammensetzung.

10. Zusammensetzung gemäß Anspruch 9, wobei die Fasern in festen Formulierungen in einem Bereich vorliegen, umfassend 5 bis 25 %.

11. Zusammensetzung gemäß Anspruch 9, wobei die Fasern in festen Formulierungen in einem Bereich vorliegen, umfassend 10 bis 20 %.

12. Zusammensetzung gemäß Anspruch 9, wobei die Fasern in flüssigen Formulierungen in einem Bereich vorliegen, umfassend 2 bis 15 %.

13. Zusammensetzung gemäß Anspruch 9, wobei die Fasern in flüssigen Formulierungen in einem Bereich vorliegen, umfassend 5 bis 10 %.

14. Zusammensetzung gemäß einem der vorherigen Ansprüche 1-13, wobei des Weiteren Probiotika, lebend oder tyndalisiert, zugegeben werden.

15. Zusammensetzung gemäß einem der vorherigen Ansprüche 1-14 in Kombination mit pharmazeutisch anerkannten Trägerstoffen und/oder Verdünnungsmitteln, die für feste Formulierungen geeignet sind, ausgewählt aus der Gruppe bestehend aus Pulvern, Brausepulvern, Kautabletten und Tabletten.

16. Zusammensetzung gemäß einem der vorherigen Ansprüche 1-14 in Kombination mit lebensmitteltauglich anerkannten Trägerstoffen und/oder Verdünnungsmitteln, die für feste Formulierungen geeignet sind, ausgewählt aus der Gruppe bestehend aus Pulvern, Brausepulvern, Kautabletten und Tabletten.

17. Zusammensetzung gemäß einem der vorherigen Ansprüche 1-14 in Kombination mit pharmazeutisch anerkannten Trägerstoffen und/oder Verdünnungsmitteln für flüssige Formulierungen, ausgewählt aus der Gruppe bestehend aus Sirups, Lösungen, Suspensionen und wässrigen Dispersionen.

18. Zusammensetzung gemäß einem der vorherigen Ansprüche 1-14 in Kombination mit lebensmitteltauglich anerkannten Trägerstoffen und/oder Verdünnungsmitteln, die für flüssige Formulierungen geeignet sind, ausgewählt aus der Gruppe bestehend aus Sirups, Lösungen, Suspensionen und wässrigen Dispersionen.

19. Verwendung von mindestens einem Phytoöstrogen selektiven Agonisten für den Östrogenrezeptor Beta und mindestens eine unlösliche und unverdauliche Faser oder Mischung daraus, ausgewählt aus der Gruppe, bestehend aus Nicht-Stärke-Polysacchariden zur Herstellung der Zusammensetzungen gemäß einem der vorherigen Ansprüche für die prophylaktische oder therapeutische lokale Behandlung von pathologischen Zuständen der Darmschleimhaut, ausgewählt aus der Gruppe bestehend aus adenomatöser Polyposis und deren Progression zu fortgeschrittenen Adenomen und Adenokarzinom mittels oraler Verabreichung.

## Revendications

1. Composition comprenant au moins un phytoestrogène sélectivement agoniste au récepteur-béta de l'estrogène et au moins une fibre insoluble et indigestible ou des mélanges de celle-ci sélectionnés dans le groupe consistant en polysaccharides non-amidon pour utilisation dans le traitement local prophylactique ou thérapeutique de conditions pathologiques de muqueuse intestinale sélectionnées dans le groupe consistant en adéno polypose et sa progression vers des adénomes et adénocarcinomes avancés par administration orale.

2. Composition selon la revendication 1, dans laquelle les phytoestrogènes, sous forme purifiée ou comme extraits, sont sélectionnés dans le groupe consistant en silymarine, silybinine, iso-silybinine, silydianine, silychristine, génistéine, resveratrol, coumestrol et leurs mélanges.

3. Composition selon la revendication 1, dans laquelle le phytoestrogène est l'extrait de Chardon-Marie de silymarine consistant en un mélange de silymarine, silybinine, iso-silybinine, silydianine, silychrisine.

4. Composition selon la revendication 1, dans laquelle les fibres insolubles et indigestibles, sous forme purifiée ou comme extraits, sont sélectionnées dans le groupe consistant en lignine, cellulose, insuline, insuline à chaîne longue et leurs mélanges.

5. Composition selon les revendications 2 à 4, dans laquelle le phytoestrogène est la silymarine et la fibre insoluble et indigestible est la lignine.

6. Composition selon les revendications 1 à 5, dans laquelle les phytoestrogènes sont dans une plage comprise entre 0,1 et 10% du poids total de ladite composition.

7. Composition selon la revendication 6, dans laquelle les phytoestrogènes sont dans une plage comprise entre 0,5 et 5% du poids total de ladite composition.

8. Composition selon la revendication 6, dans laquelle les phytoestrogènes sont dans une plage comprise entre 1 et 2% du poids total de ladite composition.

9. Composition selon les revendications 1 à 5, dans laquelle les fibres insolubles et indigestibles sont dans une plage entre 1 et 30% du poids total de ladite composition.

10. Composition selon la revendication 9, dans laquelle les fibres sont dans une plage entre 5 et 25% dans des formulations solides.

11. Composition selon la revendication 9, dans laquelle les fibres sont dans une plage comprise entre 10 et 20% dans des formes solides.

12. Composition selon la revendication 9, dans laquelle les fibres sont dans une plage comprise entre 2 et 15% dans des formes liquides.

13. Composition selon la revendication 9, dans laquelle les fibres sont dans une plage comprise entre 5 et 10% dans des formes liquides.

14. Composition selon l'une quelconque des revendications précédentes 1 à 13, dans laquelle des probiotiques, vivants ou tyndallisés sont en outre ajoutés.

15. Composition selon l'une quelconque des revendications 1 à 14, en combinaison avec des excipients et/ou diluants pharmaceutiquement acceptables qui conviennent à des formes solides sélectionnées dans le groupe consistant en poudres, poudres effervescentes, tablettes à mâcher et tablettes.

16. Composition selon l'une quelconque des revendications précédentes 1 à 14, en combinaison avec des excipients et/ou diluants acceptables de grades alimentaires qui conviennent pour les formes solides sélectionnées dans le groupe consistant en poudres, poudres effervescentes, tablettes à mâcher et tablettes.

17. Composition selon l'une quelconque des revendications 1 à 14, en combinaison avec des excipients et/ou diluants pharmaceutiquement acceptables qui conviennent pour des formes liquides sélectionnées dans le groupe consistant en sirops, solutions, suspensions et dispersions aqueuses.

18. Composition selon l'une quelconque des revendications précédentes 1 à 14, en combinaison avec des excipients et/ou diluants acceptables de grade alimentaire qui conviennent pour des formes liquides sélectionnées dans le groupe consistant en sirops, solutions, suspensions et dispersions aqueuses.

19. Utilisation d'au moins un phytoestrogène sélectivement agoniste au récepteur-béta de l'estrogène et au moins d'une fibre insoluble et indigestible ou des mélanges de ceux-ci sélectionnés dans le groupe consistant en polysaccharides non-amidon pour la préparation des compositions selon l'une quelconque des revendications précédentes pour le traitement local prophylactique ou thérapeutique de conditions pathologiques de la muqueuse intestinale sélectionnées dans le groupe consistant en adéno polypose et sa progression vers des adénomes et adénocarcinomes avancés par administration orale.
